# EUROPEAN PATENT APPLICATION

(11) **EP 1 542 017 A1**
(43) Date of publication of application: **15.06.2005**
(21) Application number: 03762879.9
(22) Date of filing: 04.07.2003
(51) Int. Cl.: G01N 33/68, G01N 33/50, G01N 30/88

(54) **METHOD OF EXAMINING AND DIAGNOSING INTEGRATION DYSFUNCTION SYNDROME**

(30) Priority: 04.07.2002 JP 2002195315
(71) Applicant: Mitsubishi Pharma Corporation, Osaka-shi, Osaka 541-0046 (JP)
(72) Inventor: HASHIMOTO, Kenji, Nishitokyo-shi, Tokyo 202-0013 (JP); IYO, Masaomi, Chiba-shi, Chiba 262-0033 (JP); FUKUSHIMA, Takeshi, Shizuoka-shi, Shizuoka 420-0816 (JP); IMAI, Kazuhiro, Setagaya-ku, Tokyo 155-0033 (JP)
(74) Representative: von Kreisler Selting Werner
(86) International application number: PCT/JP2003/008525
(87) International publication number: WO 2004/005935

(57) **Abstract**

The present invention relates to a method of examining or diagnosing schizophrenia, which includes measuring concentrations of D- serine and L- serine in a biological sample. According to the method of the present invention, schizophrenia can be examined or diagnosed by measuring concentrations of D-serine and L-serine in a biological sample by analysis techniques such as high performance liquid chromatography and the like.

## Description

### Technical Field

The present invention relates to a method of examining or diagnosing schizophrenia. More particularly, this invention relates to a method of examining or diagnosing schizophrenia, which comprises measuring the concentration of D-serine, L-serine or D-serine and L-serine in a biological sample.

### Background Art

About 1% of the population has been afflicted with schizophrenia and the disease is developed during the period of from puberty to early 20s. As the symptoms, positive symptoms such as hallucination, delusion and the like, negative symptoms such as flattened affect, loss of incentive, social withdrawal and the like, and cognitive dysfunction and the like can be mentioned. In view of the peculiarity of the disease state, establishment of a comprehensive treatment system including diagnosis and treatment in early stages, activity for reintegration into society and prevention of recurrence has been desired. For the treatment of schizophrenia, a drug treatment is indispensable and phenothiazine compounds, butyrophenone compounds, benzamide compounds, iminodibenzyl compounds, thiepin compounds, indol compounds, serotonin-dopamine receptor blockers and the like are administered.

It has been known that genetic factors and environmental factors are involved in the disease state of schizophrenia. It has been known that NMDA receptor blockers such as phencyclidine, ketamine and the like cause symptoms extremely similar to those of schizophrenia, and what is called the "glutamate hypofunction hypothesis of schizophrenia" has been proposed. At present, a biological marker to examine or diagnose schizophrenia has not been found. While many studies have been done using samples from patients such as blood, urine and the like, no biological marker has been established so far.

Incidentally, D-amino acid has been considered to be absent in mammals. However, it has been recently clarified that D-amino acid including D-serine is present also in mammals including human at a low concentration. On the other hand, D-serine is known to function as an agonist for a glycine site of NMDA receptor, which is a subtype of the glutamate receptor controlling excitatory neurotransmission. According to the glutamate hypofunction hypothesis of schizophrenia, there is a possibility that D-serine is involved in the disease state of schizophrenia.

Therefore, a method of administering D-serine for the treatment of patients with schizophrenia (US Patent No. 6162827), and a method of administering D-serine, D-alanine and the like, which are agonists for the glycine site of NMDA receptor for the treatment of neuropsychiatric diseases such as schizophrenia, Alzheimer's disease, autism, depression and the like (WO99/52519), are known.

The number of inpatients with schizophrenia occupies about 15% of the total number of beds in the hospitals in Japan, presenting a critical problem in terms of medical economy. Thus, there is a demand from those actually practicing medicine, for the development of an examination and diagnostic drug, and an examination or diagnosis method affording examination or diagnosis of schizophrenia in early stages.

Note that, in Japanese, schizophrenia was conventionally referred to as "*seishin-bunretsu-byo*" but in the annual meeting of The Japanese Society of Psychiatry and Neurology held in August 2002, the name was changed to "*togo-shiccho-sho*". Both "*seishin-bunretsu-byo*" and "*togo-shiccho-sho*" mean "schizophrenia".

### Disclosure Of The Invention

The present inventors have conducted intensive studies in an attempt to solve the above-mentioned problems and found that the levels of D-serine and L-serine in biological samples (e.g., serum etc.) from patients with schizophrenia significantly change as compared to those of healthy subjects.

Particularly, the present inventors have first found that D-serine having an NMDA receptor agonist activity shows a significant decrease in the peripheral blood of patients with schizophrenia. There have been reports on the increase in serine (total serine: total of D-serine and L-serine, about 99% being L-serine) in the peripheral blood of patients with schizophrenia. The total serine, L-serine and D-serine have been respectively measured at this time, and as a result, it has been clarified that the total serine showed an increase (P=0.017) in the patients with schizophrenia, and an increase (P=0.012) in L-serine as well. To the contrary, D-serine showed a decrease (P=0.001) in the patients with schizophrenia, and the D-serine/total serine ratio showed a distinct decrease (P=0.0001) in the patients. While no clear correlation was found between the decrease in D-serine and the disease state (negative, positive and the like) of patients, the presence or absence of drug treatment, administration background of pharmaceutical agents and the like, when a drug treatment group alone was analyzed, a positive correlation was rather found between D-serine and BPRS score (Brief Psychiatric Rating Scale, lower scores mean higher improvement). As for the decrease in D-serine, the involvement of serine racemase, which is a D-serine synthase, D-amino acid oxidaze, which is a decomposing enzyme, D-serine transporter and the like is considered.

The present invention has been completed based on the above-mentioned findings and the like.

Accordingly, the present invention relates to the following (1) to (28).
(1) A method of examining schizophrenia, which comprises measuring concentration(s) of (a) D-serine, (b) L-serine or (c) D-serine and L-serine in a biological sample.
(2) The examination method of the aforementioned (1), wherein an index is that the D-serine concentration in a biological sample is lower than an average of said concentration in a healthy individual or a group of healthy individuals.
(3) The examination method of the aforementioned (1), wherein an index is that the D-serine concentration in a biological sample is lower than an average-standard deviation of the concentration in a healthy individual or a group of healthy individuals.
(4) The examination method of the aforementioned (1), wherein an index is that the D-serine concentration in a biological sample is lower than the average + standard deviation of said concentration in an individual with schizophrenia or a group of individuals with schizophrenia.
(5) The examination method of the aforementioned (1), wherein an index is that the L-serine concentration in a biological sample is higher than an average of said concentration in a healthy individual or a group of healthy individuals.
(6) The examination method of the aforementioned (1), wherein an index is a ratio of the D-serine concentration to the total serine concentration in a biological sample.
(7) The examination method of the aforementioned (6), wherein an index is that the ratio of the D-serine concentration to the total serine concentration in a biological sample is lower than an average of said ratio in a healthy individual or a group of healthy individuals.
(8) The examination method of the aforementioned (6), wherein an index is that the ratio of the D-serine concentration to the total serine concentration in a biological sample is lower than an average-standard deviation of said ratio in a healthy individual or a group of healthy individuals.
(9) The examination method of the aforementioned (6), wherein an index is that the ratio of the D-serine concentration to the total serine concentration in a biological sample is lower than an anaverage+standard deviation of said ratio in an individual with schizophrenia or a group of individuals with schizophrenia.
(10) The examination method of any of the aforementioned (1) to (9), further comprising selecting patients with schizophrenia for whom the D-serine therapy is effective.
(11) The examination method of any of the aforementioned (1) to (10), which uses an amino acid labeling reagent.
(12) The examination method of the aforementioned (11), further comprising steps of contacting an amino acid labeling reagent with a biological sample to label serine and separating or quantifying the labeled D-serine and the labeled L-serine.
(13) The examination method of the aforementioned (12), further comprising a step of separating and quantifying the labeled serine before the step of separating or quantifying the labeled D-serine and the labeled L-serine.
(14) The examination method of the aforementioned (12), wherein the labeled D-serine and the labeled L-serine are separated or quantified using a column or capillary.
(15) The examination method of the aforementioned (13), wherein the labeled D-serine and the labeled L-serine are separated or quantified using a column or capillary.
(16) The examination method of the aforementioned (13), wherein the labeled serine is separated or quantified using chromatography.
(17) The examination method of the aforementioned (15), wherein the labeled serine is separated or quantified using chromatography.
(18) The examination method of the aforementioned (14), (15) or (17), wherein the column or capillary is a column or capillary for optical resolution.
(19) The examination method of the aforementioned (16) or (17), wherein the chromatography is high performance liquid chromatography.
(20) The examination method of the aforementioned (17), wherein the chromatography is high performance liquid chromatography and the column or capillary is a column or capillary for optical resolution.
(21) The examination method of any of the aforementioned (11) to (20), wherein the amino acid labeling reagent is an amino acid fluorescence-labeling reagent.
(22) The examination method of the aforementioned (21), wherein the amino acid fluorescence-labeling reagent is 4-fluoro-7-nitro-2,1,3-benzoxadiazole.
(23) A reagent for examining schizophrenia, which comprises an amino acid labeling reagent.
(24) The reagent of the aforementioned (23), wherein the amino acid labeling reagent is an amino acid fluorescence-labeling reagent.
(25) The reagent of the aforementioned (24), wherein the amino acid fluorescence-labeling reagent is 4-fluoro-7-nitro-2,1,3-benzoxadiazole.
(26) A method of examining or diagnosing schizophrenia, which comprises measuring concentrations of D-serine and L-serine in serum and using an increase or decrease in the concentration as an index.
(27) A method of examining or diagnosing schizophrenia, which comprises measuring a D-serine concentration in serum and using a decrease in the concentration as an index.
(28) A method of examining or diagnosing schizophrenia, which comprises measuring an L-serine concentration in serum and using an increase in the concentration as an index.

### Best Mode For Carrying Out The Invention

For example, the examination and diagnosis of schizophrenia in the present invention can be performed as in the following. A biological sample (e.g., human serum) is prepared, and the concentrations of the total serine, D-serine and/or L-serine in the biological sample are quantified by various methods. The schizophrenia is examined or diagnosed based on the fact that the D-serine concentration and/or the concentration ratio of D-serine to the total serine in a biological sample (e.g., serum) from patients with schizophrenia is significantly lower than those values of healthy subjects and the like.

Accordingly, as an index of schizophrenia, for example,
i) an index that D-serine concentration in a biological sample is lower than an average of said concentration in a healthy individual or a group of healthy individuals,
ii) an index that an L-serine concentration in a biological sample is higher than an average of said concentration in a healthy individual or a group of healthy individuals,
iii) an index that a ratio of the D-serine concentration to the total serine concentration in a biological sample is lower than an average of said ratio in a healthy individual or a group of healthy individuals,
   and the like can be mentioned.
   Moreover, as a preferable index of schizophrenia, for example,
iv) an index that a D-serine concentration in a biological sample is lower than an average-standard deviation of said concentration in a healthy individual or a group of healthy individuals,
v) an index that a D-serine concentration in a biological sample is lower than the average + standard deviation of said concentration in an individual with schizophrenia or a group of individuals with schizophrenia,
vi) an index that a ratio of the D-serine concentration to the total serine concentration in a biological sample is lower than an average-standard deviation of said ratio in a healthy individual or a group of healthy individuals,
vii) an index that a ratio of the D-serine concentration to the total serine concentration in a biological sample is lower than an anaverage+standard deviation of said ratio in an individual with schizophrenia or a group of individuals with schizophrenia,
   and the like can be mentioned.

As used herein, that the "D-serine concentration is lower than an average (-standard deviation) of said concentration in a healthy individual or a group of healthy individuals" means that the "D-serine concentration of a single individual is lower than the average (-standard deviation) of plural measures of said concentration when the individual is healthy", or that the "D-serine concentration in a single individual is lower than the average (-standard deviation) of said concentration in a group of healthy individuals". In addition, that the "D-serine concentration is lower than the average + standard deviation of said concentration in an individual with schizophrenia or a group of individuals with schizophrenia" means that the "D-serine concentration in a single individual is lower than the average (+standard deviation) of plural measures of the concentration when the individual has schizophrenia", or that the "D-serine concentration in a single individual is lower than the average (+standard deviation) of the concentration of a group of individuals having schizophrenia". In the present specification, "an average (-standard deviation) of said ratio in a healthy individual or a group of healthy individuals", "an average + standard deviation of said ratio in an individual with schizophrenia or a group of individuals with schizophrenia" and the like are to be interpreted in the same manner.

In the present invention, it is also possible to examine or diagnose schizophrenia by measuring only the D-serine concentration in a biological sample and using an index that the D-serine concentration is lower than that of a healthy individual or a group of healthy individuals. In this case, a preferable index when, for example, the biological sample is a serum, is that the D-serine concentration is not more than 2.00 µmol/L.

In the present invention, it is also possible to examine or diagnose schizophrenia by measuring only the L-serine concentration in a biological sample and using an index that the L-serine concentration is higher than that of a healthy individual or a group of healthy individuals. In this case, a preferable index when, for example, the biological sample is a serum is that the L-serine concentration is 190 µmol/L.

In the present invention, moreover, it is also possible to examine or diagnose schizophrenia by measuring the total serine concentration in a biological sample and using an index that the total serine concentration is higher than that of a healthy individual or a group of healthy individuals. In this case, a preferable index when, for example, the biological sample is a serum is that the total serine concentration is not less than 190 µmol/L.

As the biological sample that can be subjected to the examination or diagnosis of the present invention, for example, liquid components derived from living organisms such as serum, plasma, blood, urine, cerebrospinal fluid, saliva, tear, sweat and the like, solid components derived from living organisms such as hair, blood cell, partial tissue removed by biopsy etc., and the like, and the like can be mentioned, which include any component derived from living organisms. Preferable biological sample is serum. In addition, a product obtained by various treatments of a sample directly obtained from a living organism can be a biological sample in the present invention. In this case, the kinds and number of the treatments are not limited and plural kinds of treatments may be combined. As the kind of treatment, for example, there can be mentioned, but not limited to, protein elimination, desalting, dialysis, addition of acid/base/buffer and the like, heating, separation and purification of amino acid-containing fractions by a column and the like.

As the living organisms from which the samples to be subjected to the examination or diagnosis of the present invention derive, mammals inclusive of human (e.g., human, monkey, mouse, rat, guinea pig etc.) can be mentioned.

In the method of examining or diagnosing schizophrenia of the present invention, the concentrations of D- and L-serine in a biological sample can be measured by any method known *per se*. Any method to be newly developed from now on can be also used for the measurement of the concentrations of D-and L-serine in a biological sample. Preferably, amino acid including serine in a biological sample is labeled with an amino acid labeling reagent to enhance the detection sensitivity. As used herein, by the amino acid labeling reagent is meant a reagent that labels amino acid for the purpose of enhancing the detection sensitivity in the separation, quantification and the like of amino acid, wherein the amino acid labeling reagent and amino acid/serine are reacted to respectively give labeled amino acid/serine. As the amino acid labeling reagent, an amino acid absorbance-labeling reagent, an amino acid fluorescence-labeling reagent and an amino acid luminescence-labeling reagent can be mentioned, wherein reaction with amino acid/serine yields absorbance-labeled amino acid/serine, fluorescence-labeled amino acid/serine and luminescence-labeled amino acid/serine, respectively. The absorbance, fluorescence intensity or luminescence intensity is used as an index to separate or quantify amino acid, serine in a biological sample. Preferably, an amino acid fluorescence-labeling reagent is used. As the amino acid absorbance-labeling reagent, for example, ninhydrin and the like can be mentioned. As the amino acid fluorescence-labeling reagent, for example, orthophthalaldehyde (OPA), fluorescamine (FLA), naphthalene-2,3-dicarboxyaldehyde (NDA), dansyl chloride (DNS-Cl), 9-fluorenyl methylchloroformate (FMOC-Cl), 3,4-dihydro-6,7-dimethoxy-4-methyl-3-oxoquinoxaline-2-carbonylchloride (DMEQ-COCl), 7-methylcoumarin 3-carbonylfluoride, 4-fluoro-7-nitro-2,1,3-benzoxadiazole (NBD-F), 4-chloro-7-nitro-2,1,3-benzoxadiazole (NBD-Cl), DBD reagent, fluorescent Edman reagent, fluorescein isothiocyanate, DBD-NCS, DBD-Pro-COCl, NBD-Pro-COCl, (+)-1-(1-isocyanateethyl)naphthalene, (+)-1-(9-fluorenyl)ethylchloroformate , N-acetyl-L-cysteine and the like can be mentioned, each of which can be used in the present invention. Preferably, 4-fluoro-7-nitro-2,1,3-benzoxadiazole (NBD-F) is used for reaction with amino acid/serine to give NBD-labeled amino acid/serine.

As a method of measuring serine in a biological sample in the method of examining or diagnosing schizophrenia of the present invention, for example, a measurement method comprising a step of contacting an amino acid labeling reagent with a biological sample to label serine and a step of separating or quantifying the labeled D-serine and the labeled L-serine can be mentioned.

Here, it is preferable to further comprise a step of separating or quantifying the labeled serine before the step of separating or quantifying the labeled D-serine and the labeled L-serine.

In other words, a preferable measurement method of serine in a biological sample in the method of examining or diagnosing schizophrenia of the present invention comprises, for example, the following steps:
Step 1: contacting an amino acid labeling reagent with a biological sample to label serine;
Step 2: separating or quantifying the labeled serine;
Step 3: separating or quantifying the labeled D-serine and the labeled L-serine.

The measurement method is generally performed in the order of Step 1 → Step 2 → Step 3. It is not prohibited to perform different steps before Step 1, between Step 1 and Step 2, or between Step 2 and Step 3.

In the above-mentioned Step 2, a method of separating or quantifying the labeled serine is not particularly limited but chromatography is preferably used. As the chromatography usable for separation or quantification of labeled serine, for example, chromatography using a liquid mobile phase (e.g., high performance liquid chromatography (HPLC), medium-high pressure liquid chromatography etc.), chromatography using a gaseous mobile phase (e.g., gas chromatography) and chromatography using a supercritical liquid mobile phase (e.g., supercritical liquid chromatography) can be mentioned, and high performance liquid chromatography is preferably used. As the mobile phase or a sample transfer means, a physical means using a pump and the like or an electric means (e.g., electrophoresis etc.) can be also used.

A labeled serine is separated via a suitable separation medium installed in the above-mentioned chromatography device. As a preferable separation medium, column, capillary, gel, thin layer and the like can be mentioned, and a column is more preferable. As a column usable for the separation of labeled serine, for example, reversed phase column, normal phase column, ion exchange column, ligand exchange column, ion-exclusion column, size-exclusion column (GPC and GFC), column for optical resolution, affinity column and the like can be mentioned, and reversed phase column or ion exchange column is preferably used.

A separated labeled serine is quantified using a suitable detection means. The absorbance labeled serine is desirably quantified by an absorbance photometer, and a fluorescence labeled serine is desirably quantified by a fluorescence photometer. By this step, the total serine concentration of a biological sample can be measured.

In Step 2, plural kinds of chromatographys may be combined or plural kinds of separation media may be combined.

In the above-mentioned Step 3, a method of separation or quantification of the labeled D-serine and the labeled L-serine is not particularly limited but chromatography is preferably used. As the chromatography, those similar to the chromatographys usable for the separation or quantification of the labeled serine in the above-mentioned Step 2 can be exemplarily mentioned, and high performance liquid chromatography is preferably used. As the mobile phase or a sample transfer means, a physical means using a pump and the like or an electric means (e.g., electrophoresis etc.) can be also used.

The labeled D-serine and the labeled L-serine are separated via a suitable separation medium installed in the above-mentioned chromatography. Any mode of separation medium can be used as long as the object can be achieved, and column, capillary, gel and thin layer can be preferably mentioned, and column or capillary is more preferably used. As the column or capillary usable for the separation of a labeled D-serine and a labeled L-serine, for example, reversed phase column or capillary, normal phase column or capillary, ion exchange column or capillary, ligand exchange column or capillary, ion-exclusion column or capillary, size-exclusion column (GPC and GFC) or capillary, column or capillary for optical resolution, affinity column or capillary and the like can be mentioned, and column or capillary for optical resolution is preferably used.

A separated labeled D-serine and a labeled L-serine are quantified using the aforementioned suitable detection means. By this step, a D-serine concentration and an L-serine concentration in a biological sample can be measured. In Step 3, plural kinds of chromatographys may be combined or plural kinds of separation media may be combined.

As mentioned above, it is preferable to separate labeled serine in Step 2, and further separate or quantify same into labeled D-serine and labeled L-serine in Step 3. In this case, labeled serine separated in Step 2 is once obtained by separation and used in Step 3. Alternatively, Step 2 and Step 3 may be continuously performed by continuously setting plural columns in one chromatography device via a switching valve and the like.

For example, a further specific method of measuring D-serine and L-serine in a biological sample comprises a method comprising the following steps:
Step (A): contacting 4-fl-uoro-7-nitro-2,1,3-benzoxadiazole (NBD-F) with a biological sample to label serine;
Step (B): separating or quantifying the NBD-labeled serine by high performance liquid chromatography;
Step (C): separating or quantifying the NBD-labeled D-serine and NBD-labeled L-serine using a column for optical resolution;
   a method comprising the following steps:
Step (I); contacting an amino acid fluorescence-labeling reagent such as 4-fluoro-7-nitro-2,1,3-benzoxadiazole and the like with a biological sample to synthesize fluorescence-labeled amino acid (e.g., NBD-labeled serine etc.);
Step (II): separating or quantifying the NBD-labeled serine by high performance liquid chromatography;
Step (III): separating or quantifying the separated NBD-labeled serine using a column for optical resolution; and the like.

The method of examining or diagnosing schizophrenia of the present invention can be used in combination with conventional subjective examinations or diagnostic methods (e.g., BPRS score judgment according to DSM-IV etc.) or any examination or diagnostic methods to be developed from now on. In addition, a definitive judgment can be made only by the examination or diagnostic method of the present invention, by collecting the data of quantified value of D-serine and L-serine usable as indices in the examination or diagnostic method of the present invention, from patients evidently having schizophrenia by some method. The examination or diagnostic method of the present invention can be also applied to a psychiatric examination conducted to investigate the presence or absence of legal competency or for other purposes.

Using the examination or diagnostic method of the present invention, determination of the disease state (negative, positive and the like) of patients, determination of severity, process observation of disease state, determination of the presence or absence of treatment effect, prediction of prognosis, discrimination from other mental diseases and the like can be easily conducted. Particularly, since patients having particularly low concentration ratio of D-serine to the total serine can be selected, control patients for whom D-serine therapy is effective can be selected before the start of the treatment and more efficient treatment can be conducted. Here, by the D-serine therapy is meant, for example, a treatment method of schizophrenia, which increases D-serine in living organisms by the administration of D-serine itself, a compound that increases expression of serine racemase, a compound that activates serine racemase, D-serine transporter inhibitor or D-serine decomposing enzyme inhibitor and the like.

As an index based on which to select patients with schizophrenia for whom D-serine therapy is effective, the aforementioned indices for schizophrenia can be used.

Accordingly, as the index based on which to select patients with schizophrenia for whom D-serine therapy is effective, for example,
I) an index that a D-serine concentration in a biological sample is lower than an average concentration in a healthy individual or a group of healthy individuals,
II) an index that an L-serine concentration in a biological sample is higher than an average of said concentration in a healthy individual or a group of healthy individuals,
III) an index that a ratio of the D-serine concentration to the total serine concentration in a biological sample is lower than an average of said ratio in a healthy individual or a group of healthy individuals,
   and the like can be mentioned.
   As a preferable index based on which to select patients with schizophrenia for whom D-serine therapy is effective, for example,
IV) an index that a D-serine concentration in a biological sample is lower than an average-standard deviation of said concentration in a healthy individual or a group of healthy individuals,
V) an index that a D-serine concentration in a biological sample is lower than the average + standard deviation of said concentration in an individual with schizophrenia or a group of individuals with schizophrenia,
VI) an index that a ratio of the D-serine concentration to the total serine concentration in a biological sample is lower than an average-standard deviation of said ratio in a healthy individual or a group of healthy individuals,
VII) an index that a ratio of the D-serine concentration to the total serine concentration in a biological sample is lower than an anaverage+standard deviation of said ratio in an individual with schizophrenia or a group of individuals with schizophrenia, and the like can be mentioned.

From the foregoing, an amino acid labeling reagent, preferably an amino acid fluorescence-labeling reagent, more preferably 4-fluoro-7-nitro-2,1,3-benzoxadiazole can be used as a reagent for the examination or diagnosis of schizophrenia.

The method of the present invention is also useful for the determination of a "candidate substance for an anti-schizophrenia drug". Namely, a compound having an activity to increase D-serine or decrease L-serine is potentially useful as an anti-schizophrenia drug. The "candidate substance for an anti-schizophrenia drug" can be any substance desired by a test operator.

### Example

The present invention is explained in more detail in the following by referring to Experimental Examples, which are not to be construed as limitative of the scope of the present invention in any way.

### Experimental Example 1

Blood was taken from 42 healthy subjects and 42 patients with schizophrenia, and the serum was separated by a conventional method and subjected to the following experiment. All patients were diagnosed according to DSM-IV, and BPRS scores were evaluated by medical specialists. The backgrounds of the test subjects are as shown in Table 1.

### (Test Method)

Methanol (180 µL) was added to 20 µL of human serum to eliminate protein and the mixture was centrifuged. To the supernatant (20 µL) was added 20 µL of 0.1 M borate buffer (pH 8.0) and 60 µL of 50 mM NBD-F solution in acetonitrile, and the mixture was heated at 60°C for 1 min. Thereto was added a water/acetonitrile (90/10) mixture (200 µL) containing 0.1% trifluoroacetate. The solution (10 µL) was injected into HPLC.

For HPLC, an ODS column (TSKgel ODS-80Ts, Tosoh) and chiral column (Sumichiral OA-2500(S), Sumika Analytical Center) were connected with a six-port valve and used for column-switching HPLC. Thus, the NBD-labeled serine (total of NBD-labeled D-serine and NBD-labeled L-serine) was separated and quantified with the ODS column, and subsequently, the NBD-labeled D-serine and the NBD-labeled L-serine were separated and quantified with a chiral column. For the detection and quantification of the NBD-labeled serine, a fluorescence detector (Hitachi) was used. The excitation wavelength was 470 nm and the emission wavelength was 530 nm.

For separation and quantification of NBD-labeled serine with an ODS column, the mobile phase was flowed at a constant flow rate of 0.8 mL/min. As the mobile phase, solution A (water/acetonitrile (90/10) mixture containing 0.1% trifluoroacetate), solution B (water/acetonitrile (10/90) mixture containing 0.1% trifluoroacetate) and solution C (acetonitrile) were prepared. The elution conditions were as follows.
0-25.0 min solution A:solution B:solution C=92:8:0
25.1-35.0 min solution A:solution B:solution C=0:100:0
35.1-45.0 min solution A:solution B:solution C=0:0:100

For separation and quantification of NBD-labeled D-serine and NBD-labeled L-serine with a chiral column, the mobile phase was flowed at a constant flow rate of 0.8 mL/min. As the mobile phase, a 15 mM citric acid solution in methanol was used.

For quantification of NBD-labeled D-serine and NBD-labeled L-serine, standard compounds (Sigma) of D-serine and L-serine were treated in the same manner as above, and calibration curves were formed. The fluorescence peak area obtained when each sample was treated was plotted against the calibration curve, and the D-serine concentration and the L-serine concentration were calculated.

By the above operations, D-serine and L-serine in human sera were quantified.

### (Results)

In the healthy subject group and schizophrenia group, the contents of D-serine and L-serine in human sera were measured by high performance liquid chromatography and the concentrations were calculated. The results are shown in Table 1.

**Table 1**

| | healthy subject group n=42, M/F=21/21 mean±S.D. | schizophrenia group n=42, M/F=22/20 mean±S.D. | P value |
|---|---|---|---|
| Age, year | 35.5±14.4(20-7D) | 36.0±14.7(16-75) | 0.932 |
| Onset, year | | 25.3±11.6(13-57) | |
| Duration of illness (years) | | 10.2±11.0(0-41) | |
| BPRS scores | | | |
| Total | | 23.1±14.5(2-58) | |
| Positive | | 13.3±9.85(2-37) | |
| Negative | | 5.12±4.02(0-16) | |
| Total serine (µmol/L) | 177.3±30.7 | 199.7±46.6 | 0.017 |
| D-serine (µmol/L) | 2.28±0.59 | 1.86±0.53 | 0.001 |
| L-serine (µmol/L) | 175.0±30.6 | 197.9±46.4 | 0.013 |
| Ratio (%) | | | |
| D-serine/total serine | 1.34±0.34 | 0.95±0.26 | <0.0001 |

In the schizophrenia group, the level of D-serine was average 1.86 µmol/L, and the level of D-serine was average 2.28 µmol/L in healthy subjects. The values of these two groups were analyzed by a non-parametric Mann-Whitney U-test. As a result, a significant (z = -4.78, p = 0.001) decrease was found in the schizophrenia patient. In addition, the D-serine concentration did not show a significant change between untreated patients and patients under treatment. As a result of the statistical analysis of patients under treatment based on chlorpromazine, the absence of correlation with D-serine concentration and the absence of correlation with illness duration were found. In the schizophrenia group, the level of L-serine was average 197.9 µmol/L, and the level of L-serine was 175.0 µmol/L in healthy subjects. The values of these two groups were analyzed by a non-parametric Mann-Whitney U-test. As a result, a significant (z = -2.49, p = 0.013) increase was found in the schizophrenia patient. In the schizophrenia group, the ratio of D-serine to the total serine was average 0.95%, and the ratio of D-serine to the total serine was average 1.34% in healthy subjects. The values of these two groups were analyzed by a non-parametric Mann-Whitney U-test. As a result, a significant (z = -4.78, p < 0.0001) decrease was found in the schizophrenia patient.

### Industrial Applicability

According to the method the present invention, whether or not the test subject is afflicted with schizophrenia can be objectively examined or diagnosed with the concentrations of D-serine and L-serine in serum as indices.

This application is based on application No. 2002-195315 filed in Japan, the contents of which are incorporated hereinto by reference.

## Claims

1. A method of examining schizophrenia, which comprises measuring concentration(s) of (a) D-serine, (b) L-serine or (c) D-serine and L-serine in a biological sample.

2. The examination method of claim 1, wherein an index is that the D-serine concentration in a biological sample is lower than an average of said concentration in a healthy individual or a group of healthy individuals.

3. The examination method of claim 1, wherein an index is that the D-serine concentration in a biological sample is lower than an average-standard deviation of the concentration in a healthy individual or a group of healthy individuals.

4. The examination method of claim 1, wherein an index is that the D-serine concentration in a biological sample is lower than the average + standard deviation of said concentration in an individual with schizophrenia or a group of individuals with schizophrenia.

5. The examination method of claim 1, wherein an index is that the L-serine concentration in a biological sample is higher than an average of said concentration in a healthy individual or a group of healthy individuals.

6. The examination method of claim 1, wherein an index is a ratio of the D-serine concentration to the total serine concentration in a biological sample.

7. The examination method of claim 6, wherein an index is that the ratio of the D-serine concentration to the total serine concentration in a biological sample is lower than an average of said ratio in a healthy individual or a group of healthy individuals.

8. The examination method of claim 6, wherein an index is that the ratio of the D-serine concentration to the total serine concentration in a biological sample is lower than an average-standard deviation of said ratio in a healthy individual or a group of healthy individuals.

9. The examination method of claim 6, wherein an index is that the ratio of the D-serine concentration to the total serine concentration in a biological sample is lower than an anaverage+standard deviation of said ratio in an individual with schizophrenia or a group of individuals with schizophrenia.

10. The examination method of any of claims 1 to 9, further comprising selecting patients with schizophrenia for whom the D-serine therapy is effective.

11. The examination method of any of claims 1 to 10, which uses an amino acid labeling reagent.

12. The examination method of claim 11, further comprising steps of contacting an amino acid labeling reagent with a biological sample to label serine and separating or quantifying the labeled D-serine and the labeled L-serine.

13. The examination method of claim 12, further comprising a step of separating and quantifying the labeled serine before the step of separating or quantifying the labeled D-serine and the labeled L-serine.

14. The examination method of claim 12, wherein the labeled D-serine and the labeled L-serine are separated or quantified using a column or capillary.

15. The examination method of claim 13, wherein the labeled D-serine and the labeled L-serine are separated or quantified using a column or capillary.

16. The examination method of claim 13, wherein the labeled serine is separated or quantified using chromatography.

17. The examination method of claim 15, wherein the labeled serine is separated or quantified using chromatography.

18. The examination method of claim 14, 15 or 17, wherein the column or capillary is a column or capillary for optical resolution.

19. The examination method of claim 16 or 17, wherein the chromatography is high performance liquid chromatography.

20. The examination method of claim 17, wherein the chromatography is high performance liquid chromatography and the column or capillary is a column or capillary for optical resolution.

21. The examination method of any of claims 11 to 20, wherein the amino acid labeling reagent is an amino acid fluorescence-labeling reagent.

22. The examination method of claim 21, wherein the amino acid fluorescence-labeling reagent is 4-fluoro-7-nitro-2,1,3-benzoxadiazole.

23. A reagent for examining schizophrenia, which comprises an amino acid labeling reagent.

24. The reagent of claim 23, wherein the amino acid labeling reagent is an amino acid fluorescence-labeling reagent.

25. The reagent of claim 24, wherein the amino acid fluorescence-labeling reagent is 4-fluoro-7-nitro-2,1,3-benzoxadiazole.

26. A method of examining or diagnosing schizophrenia, which comprises measuring concentrations of D-serine and L-serine in serum and using an increase or decrease in the concentration as an index.

27. A method of examining or diagnosing schizophrenia, which comprises measuring a D-serine concentration in serum and using a decrease in the concentration as an index.

28. A method of examining or diagnosing schizophrenia, which comprises measuring an L-serine concentration in serum and using an increase in the concentration as an index.
